## Europäisches Patentamt
## European Patent Office
## Office européen des brevets

(19)

(11) Veröffentlichungsnummer : **0 076 340**
**B1**

(12)

# EUROPÄISCHE PATENTSCHRIFT

(45) Veröffentlichungstag der Patentschrift :
**15.08.84**

(51) Int. Cl.³ : **A 23 L   2/40**, A 61 K   9/46

(21) Anmeldenummer : **81201105.4**

(22) Anmeldetag : **06.10.81**

(54) **Verfahren zur Herstellung von gewünschtenfalls zu Brausetabletten verarbeitbaren Brausegranulaten.**

(43) Veröffentlichungstag der Anmeldung :
**13.04.83 Patentblatt 83/15**

(45) Bekanntmachung des Hinweises auf die Patenterteilung : **15.08.84 Patentblatt 84/33**

(84) Benannte Vertragsstaaten :
**BE CH DE FR GB IT LI LU NL**

(56) Entgegenhaltungen :
**DE-A- 1 915 509**
**FR-A- 1 484 202**
**FR-A- 2 132 521**

(73) Patentinhaber : **Gergely, Gerhard, Dr.**
**Gartengasse 8**
**A-1050 Wien (AT)**

(72) Erfinder : **Gergely, Gerhard, Dr.**
**Gartengasse 8**
**A-1050 Wien (AT)**

(74) Vertreter : **Petraz, Gilberto Luigi**
**G.L.P. S.a.s. di Gilberto Petraz P.le Cavedalis 6/2**
**I-33100 Udine (IT)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

## Beschreibung

Vorliegende Erfindung bezieht sich auf ein Verfahren zur Herstellung von gewünschtenfalls zu Brausetabletten verarbeitbaren Brausegranulaten.

Es ist bekannt (DE-A-1 915 509/FR-A-1 484 202/FR-A-2 132 521), Brausemischungen und Brausegranulatmischungen dadurch herzustellen, dass man organische Säuren, wie Zitronensäure oder Weinsäure, mit organischen, Kohlenoxyd abspaltenden Substanzen, wie Natriumhydrogencarbonat, Natriumcarbonat, Erdalkalicarbonaten, bei höheren Temperaturen teilweise miteindander reagieren lässt und die so erhaltenen Mischungen trocknet und zerkleinert. In all diesen Fällen finden unkontrollierte Reaktionen statt, welche die Bildung der verschiedensten Alkali- oder Erdalkaliverbindungen der organischen Säure zur Folge haben. Durch diese unkontrollierten Zusammensetzungen ist auch die Lagerbeständigkeit dieser Produkte unterschiedlich und nicht optimal. So kommt es beispielsweise immer wieder bei der Lagerung der Produkte in warmen Lagerräumen oder in tropischen Gegenden zu einer weiteren an der Oberfläche der Säure ablaufenden Reaktion mit dem in der Mischung enthaltenen Carbonatbestandteil unter Abspaltung von Wasser, welches seinerseits eine weitere Reaktion der genannten Bestandteile auslösst, sodass die Umwandlung des sauren Bestandteiles in das Alkalisalz fortschreitet. Im Falle der Verwendung von Natriumcarbonat als alkalische Komponente wird diese Reaktion insoferne noch mehr begünstigt, als das Natriumbicarbonat sich bei sehr geringen Mengen von Restfeuchtigkeit thermischzersetzt und allein durch die Wärmeeinwirkung auf solche Gemische oder Tabletten bei Temperaturen ab 60 °C, soferne geringe Restfeuchtigkeitsmengen vorhanden sind, eine Kettenreaktion eintritt. Zusammen mit dieser unter Wasserbildung ablaufenden Reaktion werden natürlich Begleitstoffe, wie Salze, Farbstoffe, Aromastoffe oder Vitamine chemisch angegriffen und in vielen Fällen sogar zerstört. Solche Reaktionen können nicht nur durch die vorgenannte thermische Zersetzung von in den Brausemischungen vorhandenen Bestandteillen, wie z. B. des Natriumbicarbonats ausgelöst werden, sondern auch durch die den Bestandteilen der Mischungen anhaftende Restfeuchtigkeit, die nie völlig entfernt werden kann. Eine weitere Rolle spielt auch die Luftfeuchtigkeit, deren Auswirkung bei nicht sorgfältig dicht verpackten Produkten, oder bei der Lagerung vor der Verpackung nie auszuschliessen ist.

Brausegranulate wurden bisher in der Weise hergestellt, dass Säure und Carbonate und/oder Bicarbonate miteinander vermischt und bei höheren Temperaturen (60-100 °C) zur Reaktion gebracht werden. Diese an der Oberfläche ablaufende Reaktion führt zu zusammengepackten Agglomeraten, die sodann gemahlen und zur Erzeugung von Brausetabletten verwendet werden.

Ein grosser Nachteil dieser Verfahrensweise besteht darin, dass auf diese Weise Restfeuchtigkeit nicht völlig entfernt werden kann und vor allem auch darin, dass die bei solchen Reaktionen auftretenden Verhältnisse nicht kontrolliert werden können, sodass es stets zu Brausegemischen unterschiedlicher Zusammensetzung kommt, deren Stabilität eben aus diesem Grund mehr oder weniger gut ist.

Demgegenüber ist durch das erfindungsgemässe Verfahren die Herstellung von Brausetabletten bzw. Brausegranulaten möglich, welche nicht nur eine ausgezeichnete Lagerungsstabilität besitzen, sondern auch in ihrer Qualität immer gleichbleibend sind, da die Reproduzierbarkeit des Verfahrens durch Kontrolle einer Reihe von Reaktionsparametern jederzeit gewährleistet ist.

Die Erfindung bezieht sich demnach auf ein Verfahren zur Herstellung von gewünschtenfalls zu Brausetabletten verarbeitbaren Brausegranulaten durch Wärmebehandlung bei 30 bis 100 °C des aus Säure und Hydrogencarbonat und/oder Carbonat als Brausebestandteile bestehenden pulverförmigen oder körnigen Gemisches in einem geschlossenen System im Vakuum, wobei die Säure gegebenenfalls nach einer Vorbehandlung bei erhöhter Temperatur mit der nötigen Menge an Hydrogencarbonat und/oder Carbonat vermischt wird und man hierauf dieses Gemisch einer Vakuumbehandlung bei einer Temperatur von 30 bis 100 °C, vorzugsweise 40 bis 80 °C, bei welcher es mit einem polaren Lösungsmittel wie Wasser, Methanol, Äthanol oder Mischungen hievon versetzt wird, aussetzt, wonach die erhaltenen Aggregate zur gewünschten Teilchengrösse zerkleinert, gegebenenfalls mit den gewünschten Zusatzstoffen versehen und gegebenenfalls tablettiert werden, welches dadurch gekennzeichnet ist, dass man zur Passivierung der Oberfläche von zumindest einer der Reaktionskomponenten bzw. zur Überführung derselben in einen Zustand hoher Reaktionsträgheit während der Vakuumbehandlung eine dosierte Menge von bis zu 7 Masse-%, bezogen auf die Masse des Gemisches des polaren Lösungsmittels zum Gemisch hinzufügt, die durch die $CO_2$-Entwicklung ab dem Lösungsmittelzusatz während der Reaktion bis zum Erreichen von max. 1 000 mbar verursachte Druckdifferenz feststellt, aus dieser Druckdifferenz das Volumen und die Masse des freigesetzten $CO_2$ ermittelt und die Wärmebehandlung jeweils nach Vakuumschnelltrocknung des Gemisches so oft wiederholt, bis die durch deutliche Verlangsamung der Reaktion bzw. verminderte Gasentwicklung angezeigte Oberflächenpassivierung erreicht ist.

Die Temperatur, bei welcher das erfindungsgemässe Verfahren durchgeführt wird ist nicht kritisch und liegt aus zweckmässigkeitsgründen vorzugsweise zwischen 40 und 80 °C. Der Unterdruck, bei dem gearbeitet wird, soll möglichst nieder sein. Beispielsweise kann das Anfangsvakuum 10 mbar betragen. Das polare Lösungsmittel ist vorzugsweise Wasser, das vorzugsweise in einer Menge von 0,2-2 Masse-%, bezogen auf das Gewicht des zu behandelnden Gemisches, eingesetzt wird.

Bei der Druchführung des erfindungsgemässen Verfahrens hat es sich gezeigt, dass bei Anwendung geeigneter Konzentrationen der Reaktionspartner und geeigneter Mengen an polarem Lösungsmittel nur ein Teil der Reaktionspartner reagiert hat und dass alsbald nach Ablauf von 10-20 min die Reaktion deutlich langsamer wird. Wird nun das so zur Reaktion gebrachte und langsam weiter gerührte Gemisch schlagartig getrocknet, dann kann dieser Vorgang unter geeigneten Bedingungen wiederholt werden, wobei es sich bei der Wiederholung herausstellt, dass der genannte Vorgang verlangsamt ist, weil ein grosser Teil der Oberfläche der Säure bereits durch Alkali oder Erdalkalisalze passiviert und reaktionsträg gemacht wurde. Wenn nun dieselbe Reaktion ein drittesmal durchgeführt wird, dann kann man feststellen, dass fast keine Reaktion mehr stattfindet, da die gesamte Oberfläche bereits passiviert ist und die einzelnen Reaktionspartner in sich abgepuffert sind. Es ist also eine reaktionsträge Mischung entstanden, die selbst in Berührung mit geringen Mengen an polaren Lösungsmitteln nicht mehr oder nur sehr träge reagiert.

Es liegt auf der Hand, dass die hergestellten Mischungen im trockenen Zustand eine sehr hohe Stabilität auch bei höheren Temperaturen aufweisen, sodass Zutritt von Luftfeuchtigkeit oder lange Lagerung bei höheren Temperaturen keine weiteren Reaktionen hervorrufen können.

Da erfindungsgemäss die freigesetzte Menge $CO_2$ festgestellt wird, kann man im Zuge einfacher stöchiometrischer Berechnungen ermitteln, wieviel Bicarbonat verbraucht bzw. wieviel Salz mit der Säure gebildet wurde. Man hat es nun in der Hand, durch geeignete Parameter wie Rührgeschwindigkeit, eingesetzte Feuchtigkeitsmenge, Korngrösse der eingesetzten Säure zu bestimmen, wieviel Säure an der Oberfläche derselben zum Säuresalz umgesetzt wurde.

Bringt man beispielsweise in einem verschlossenen 100 l-Gefäss 40 kg einer Reaktionsmischung zur Reaktion, dann wird der freie Raum zwischen Masse und Kesselinhalt je nach Schüttgewicht der Masse etwa 50 l betragen. Hat man das Gefäss vor der Reaktion evakuiert, dann werden diese 50 l freier Raum praktisch gasfrei sein. Lässt man nun eine mengenmässig determinierte Menge Feuchtigkeit unter gleichzeitigem Rühren in die Masse einströmen, dann wir das entsprechende Kohlendioxyd den überstehenden Raum von 50 l ausfüllen. Diese Befüllung des Raumes lässt sich naturgemäss sehr leicht mit einem guten Vakuumeter überprüfen. Hat also das Anfangswakuum 10 mbar betragen und ist durch die Reaktion das Vakuum auf Atmosphärendruck abgefallen, dann kann man mit Sicherheit behaupten, dass etwa 50 l Kohlendioxyd entwickelt wurden.

Es lässt sich nun leicht zurückrechnen, wieviel Natriumhydrogencarbonat in diesem Falle verbraucht bzw. wieviel Natriumcitrat hergestellt wurde. Entsprechenden der Molmasse von Natriumhydrogencarbonat von 84 werden pro 84 g Natrium-hydrogencarbonat 22,4 l auf Normalbedingungen reduziertes $CO_2$ entwickelt, es sind also bei dieser Reaktion 2,23 Mole entsprechend 187,3 g Natriumhydrogencarbonat verbraucht worden und zu Natriumcitrat umgesetzt worden.

Die mehrmalige Wiederholung der erfindungsgemäss vorgesehenen Vakuumbehandlung führt fast zum Stillstand der Reaktion zwischen den Reaktionspartnern. Auch bei Gegenwart polarer Lösungsmittel und trotz der geringen Mengen an umgesetzten Materialen lässt sich kaum mehr eine Reaktion feststellen, sodass nach endgültiger Trocknung des Produktes eine ausserordentlich widerstandsfähige, aber immer noch reaktionsfähige Masse erhalten wurde. Die Ergebnisse sind bei Gleichhaltung der vorgenannten Parameter überdies streng reproduzierbar und gewährleisten die Gewinnung eines Produktes von immer gleichbleibender Qualität.

Als theoretische Erklärung für den durch das erfindungsgemässe Verfahren erzielten Effekt kann vorgebracht werden, dass die Berührungsflächen der Reaktionspartner eine Pufferzone bilden, die durch verschiedene Alkali — der Erdalkalisalze der entsprechenden Säure gebildet werden. Diese Pufferzonen werden sich selbstverständlich je nach der Oberfläche der einzelnen Kristalle und nach der Reaktionsfähigkeit ausbilden. Im Falle der Verwendung von Zitronensäure und Natriumhydrogencarbonat und/oder Natriumcarbonat als Brausekomponenten verhält es sich wahrscheinlich so, dass, obgleich Mono- und Dinatriumcitrat normalerweise mit 1 Mol Kristalwasser kristallisieren, wenn man sie nach der Reaktion im Vakuum schlagartig bei 60-70 °C trocknet, ein grosser Teil der Oberfläche der gebildeten Natriumsalze wasserfrei vorliegt.

Die Erfindung wird durch die folgenden Beispiele nähere erläutert.

Beispiel 1

In einem 100 l Vakuumkessel wurden 30 kg Zitronensäure unter Rühren 5 min lang auf 60 °C erhitzt, wonach zur Kontrolle der Restfeuchtigkeit kurz auf 20 mbar evakuiert wurde. Nach dem Aufheben des Vakuums wurden 10 kg Natriumhydrogencarbonat hinzugefügt, worauf unter weiterem Rühren abermals auf 60 °C erhitzt wurde.

Das Gemisch bestehend aus Natriumhydrogencarbonat und Zitronensäure hatte ein Schüttgewicht von annähernd 1 250 kg/m³, sodass der im Vakuumkessel verbleibende Raum etwa 50 l betrug.

Es wurde sodann auf 10 mbar evakuiert, von der Pumpe mittels Ventil abgesperrt und unter Rühren eine Menge von 210 ml Wasser eingebracht. Bei der nun einsetzenden Reaktion zwischen der Zitronensäure und dem Hydrogencarbonat unter Bildung einer passivierenden Oberflächenschicht auf den Säurekristallen und Entwicklung von $CO_2$ stieg der Druck im Vakuumkessel von 10 mbar auf 1 000 mbar an, was einem Volumen an freigesetztem $CO_2$ von 50 l bzw. bei einem Molvolumen des $CO_2$ bei 60 °C von 27,1 l einer Masse an freigesetztem $CO_2$ von 81 g entspricht.

Daraus resultiert, dass bei dieser ersten Vakuumbehandlung 36,5 g Zitronensäure an der Oberfläche zu Mononatriumcitrat umgesetzt wurden. Die Reaktionszeit betrug 4 min.

Nunmehr wurde das behandelnde Gut getrocknet und die Vakuumbehandlung unter denselben Bedingungen unter Einsatz von 300 ml Wasser wiederholt. Dabei lässt man den Druck im Vakuumkessel wieder auf 1 000 mbar ansteigen, entsprechend einem Volumen von 50 l bzw. einer Masse von 81 g $CO_2$.

Aufgrund der in der ersten Behandlung erzielten Teilweisen Passivierung der Oberfläche der Zitronensäure was diesmal die Reaktionszeit wesentlich länger und betrug etwa 10 min.

Das behandelte Gut wurde wieder getrocknet und einer dritten Vakuumbehandlung unter denselben Bedingungen unterworfen, wobei durch den unbedeutenden Druckanstieg angezeigt wurde, dass im wesentlichen keine $CO_2$-Entwicklung mehr stattfand und die Oberfläche der Säurekristalle weitestgehend passiviert war. Das Produkt wurde sodann getrocknet.

Die erhaltenen Agglomerate können sodann zur gewünschten Teilchengrösse zerkleinert werden und mit den gewünschten Zusätzen wie Aromastoffe, Vitaminen, Süssstoffen u. dgl. versetzt werden. Dieses Brausegranulat kann gegebenenfalls in an sich bekannterweise zu Tabletten verpresst werden. Das auf diese Weise erhaltene Produkt hatte auch bei tropischen Temperaturen eine ausgezeichnete Lagerfähigkeit über lange Zeitperioden.

Beispiel 2

Die Arbeitsweise gemäss Beispiel 1 wurde wiederholt, wobei jedoch bei der ersten Vakuumbehandlung nach dem Erwärmen des Gemisches auf 60 °C und Evakuieren des Vakuumkessels auf 10 mbar das Wasser in einzelnen Anteilen in einer Gesamtmenge zugesetzt wurde, bis der Druck im Behandlungsgefäss durch die während der Reaktion stattfinden $CO_2$-Entwicklung auf 1 bar, also auf Normaldruck, angestiegen war. Die Reaktionszeit betrug 10 min.

Die freigesetzte Kohlensäuremenge entsprach in diesem Falle wieder 50 l bzw. 1,84 Mol entsprechend 81 g.

Sodann wurde nach dem Trocknen des Gemisches die Behandlung mit derselben Wassermenge die nunmehr, da keine über das Ausmass der ersten Reaktion hinausgehende Reaktion auf Grund der teilweisen Passivierung der Oberfläche der Säure zu erwarten ist, sofort zur Gänze zugesetzt werden kann, wiederholt.

Der Druckanstieg betrug nach Ende der Reaktion 1 000 mbar, entsprechend einer Masse von 81 g freigesetztem $CO_2$. Die Reaktionszeit betrug 6 min.

Nach dem Trocknen wurde das Gemisch einer weiteren Vakuum behandlung unter Einsatz derselben Wassermenge und unter denselben Bedingungen unterworfen. Der unbedeutende Druckanstieg zeigte die im wesentlichen vollständige Passivierung der Oberfläche der Zitronensäurekristalle an.

Beispiel 3

Die Arbeitsweise der Beispiel 1 und 2 wurde unter Einsatz von 30 kg Weinsäure und 5 kg Natriumhydrogencarbonat unter Erzielung ähnlicher Ergebnisse wiederholt.

Beispiel 4

Besonders vorteilhaft lässt sich die Methode anwenden, wenn es darum geht, träge reagierende Substanzen in die Oberfläche von Säuren einzubauen.

30 kg Zitronensäure werden in einem 100 l Vakuumgefäss gemeinsam mit 10 kg Calciumcarbonat kleinster Korngrösse vermischt und bei 60 °C evakuiert. Es werden unter starkem Rühren 500 ml Wasser eingesaugt und das Ventil zur Vakuumpumpe geschlossen. Man rührt langsam weiter und lässt das Calciumcarbonat mit der Oberfläche von Zitronensäure reagieren. Hiebei wird ein Teil der Zitronensäureoberfläche in Monocalciumcitrat partiell wasserfrei verwandelt und gleichzeitig damit werden aber die nicht reagierten Teilchen von Calciumcarbonat an der Oberfläche von Zitronensäure verankert.

Wiederum wird bei der Auffüllung von 50 l 1,85 Mole umgesetzt, das entspricht 166 g Calciumcarbonat. Es werden also 36,5 g der Zitronensäureoberfläche in Monocalciumcitrat umgesetzt.

Wiederholt man diesen Vorgang, dann ergeben sich bereits Reaktionszeiten von 15 min., um nochmals dieselbe Menge umzusetzen.

Das vorteilhafte an diesem Verfahren ist, dass sich ein derartiges Granulat, auch wenn zur Tablette gepresst, ohne Zusatz von Natriumbicarbonat auflösst, da die Reaktionsgeschwindigkeit beim Einbringen in Wasser durch den unmittelbaren Kontakt des Calciumcarbonat eingebettet an der Zitronensäureoberfläche stark beschleunigt wird.

Beispiel 5

Um besonders schnell zu Granulaten zu gelangen, kann man Calciumcarbonat zugleich mit Natriumcarbonat zur Reaktion bringen.

In dem 100 l Vakuumkessel werden 30 kg Zitronensäure und 8 kg Calciumcarbonat und 2 kg Natriumcarbonat eingebracht. Implodiert man nun bei 60° in das System Wasser, dann wird nach dem Massenwirkungsgesetz das stärker alkalische Calciumcarbonat zunächst reagieren, die Reaktion verläuft langsam wie beim vorhergehenden Beispiel, zugleich aber wird unreagiertes Natriumbicarbonat in die Oberfläche der Zitronensäurekristalle eingebaut. Stöchiometrisch verläuft der Vorgang ungefähr wie bei dem vorgangenen Beispiel.

Wird der Vorgang wiederholt und das Granulat getrocknet, dann wird sich eine sehr starke Geschwindigkeitserhöhung der Auflösung ergeben, da das durch das gebildete Calciummonocitrat ebenfalls an die Oberfläche der Zitronensäure angeklebte Natriumbicarbonat beim Einbringen in Wasser schneller reagieren wird. Bei Kontakt eines Granulatkornes mit sehr viel Wasser wird das Massenwirkungsgesetz natürlich nicht wirksam und es wird eine individuelle Reaktion zwischen Natriumbicarbonat/Zitronensäure einerseits und Calciumcarbonat/Zitronensäure andererseits erfolgen. Das Massenwirkungsgesetz ist demnach nur wirksam, wenn es sich um geringe Flüssigkeitsmengen handelt, wie es bei der vorerwähnten Reaktion der Fall ist, da hier nur die stärkere Alkalinität des Calciumcarbonats wirksam wird.

Beispiel 6

In ebenderselben Weise können Magnesiumoxyde und Magnesium carbonate tunlichst unter gleichzeitiger Anwesenheit von Natriumbicarbonaten und Natriumcarbonaten eingesetzt werden. Auf diese Weise ergeben sich vielfältigste Möglichkeiten, von denen eine hier geschildert werden soll.

30 kg Zitronensäure werden mit 1 kg Magnesiumoxyd, 5 kg Calciumcarbonat und 2 kg Natriumcarbonat und 1 kg Kaliumbicarbonat vermischt. Bei 60 °C wird zum Zwecke der Entfernung der Restfeuchtigkeit evakuiert und eine Menge von 300 ml Wasser implodiert.

Hier entstehen bereits die mannigfaltigsten Reaktionen und man wird wie in den vorhergehenden Beispielen den Vorgang der Oberflächenpassivierung zweimal fortführen. In all diesen Fällen wird bei Auffüllen des restlichen Volumens stets eine Masse von 81 g Kohlendioxyd frei bzw. bei dreimaliger Fortführung die dreifache Menge. Es wird im einzelnen schwer feststellbar sein, von welchem der teilnehmenden Partner die Kohlensäure stammt, da das Magnesiumoxyd natürlich ebenfalls Einfluss auf die Oberflächenreaktion hat. Als Endergebnis wird aber jedenfalls ein Granulat vorliegen, an dem sowohl Natriumcarbonat als auch Kaliumbicarbonat, Magnesiumoxyd und Calciumcarbonat durch die oberflächengebildeten Salze an der Oberfläche der Zitronensäure verankert sein werden. Beim Einbringen solcher Granulate in Wasser wird sich daher eine unmittelbare Reaktion in sehr rascher Weise erzielen lassen.

Beispiel 7

Obwohl der Vorteil vorliegender Beispiele darin besteht, dass in unklimatisierten Räumen bei normaler Luftfeuchtigkeit wasserempfindliche Brausetabletten ohne besondere Kautelen mit Granulation von Wasser hergestellt werden können, kann sich manchmal die Notwendigkeit ergeben, die Reaktionsgeschwindigkeiten herabzusetzen, besonders dann, wenn es sich um sehr empfindliche und hoch spezifische Produkte handelt.

In diesem Falle kann man die Polarität des Wassers herabsetzen, indem man Alkohole vorzugsweise Methyl-Äthyl- oder Propylalkohol ein setzt und sogar ternäre Gemische vorsieht, in dem beispielsweise ein Teil Wasser mit einem Teil Äthanol und 20 Teilen Chloroform ein Reaktionspartner vorliegt, der wesentlich langsamer reagiert, als es Wasser allein wäre.

Ein Beispiel für herabgesetzte Reaktionen wird in etwa folgendermasser aussehen :

30 kg Zitronensäure werden im 100 l Vakuumgefäss evakuiert und bei 60 °C durch Implosion mit 500 ml Äthanol + 200 ml Chloroform versetzt.

Zu dieser breiig gewordenen Masse werden wie in den anderen Beispielen 10 kg Natriumhydrogencarbonat hinzugefügt und aufgrund des Dampfdruckes des Äthanols nur auf 200 mbar evakuiert. Lässt man in diese vorbehandelte Mischung, die kaum reagiert, 500 ml Wasser implodieren, dann wird eine anfänglich sehr schnelle, aber doch rasch abklingende Reaktion einsetzen. Durch die geringere Evakuierung auf 200 mbar wird im entsprechenden Verhältnis die Entwicklung von $CO_2$ geringer sein, wenn sich das Volumen der Mischung auf 1 000 mbar gasmässig ausgedehnt hat. Roh geschätzt wird der Anteil der gebildeten Natriumcitrate 80 % der unter den anderen Beispielen angeführten Menge betragen.

Wiederholt man diese Reaktion mehrere Male, dann wird man feststellen, dass das Abklingen nach mehrmaligen Behandlungen geringer ist, als es mit Wasser allein der Fall wäre. Es ist daher nötig bei dem zweiten und dritten Schritt die Gesamtflüssigkeitsmenge pro Schritt um 20 % herabzusetzen.

Solche Granulate ergeben ganz ausserordentliche Härten und sind vorzugsweise für Festgranulate, die nicht verpresst werden sollen, geeignet.

Prüft man derartig hergestellte Granulate im Gaschromatographen, dann kann man feststellen, dass ein Teil des Alkohols in der Zitronensäure gelöst bleibt und durch ein Vorhandensein eine Plastizität der Zitronensäure hervorruft. Diese in der Grössenordnung von 0,1 bis 0,01 % vorliegenden Alkoholmengen sind physiologisch unbedeutend, haben aber bei solchen Granulaten einen extremem Erweichungscharakter und daher Granulatformungscharakter für Zitronensäure.

Nur durch die Vakuumtechnologie ist es möglich, diesen Restgehalt an Lösungsmittel auf ein Minimum zu bringen, da bei ähnlichen Methoden unter Normaldruck eine weitgehende Entfernung von Alkoholen oder chlorierten Kohlenwasserstoffen überhaupt unmöglich ist.

**Ansprüche**

1. Verfahren zur Herstellung von gewünschtenfalls zu Brausetabletten verarbeitbaren Brausegranulaten durch Wärmebehandlung bei 30 bis 100 °C des aus der Säure und Hydrogencarbonat und/oder Carbonat als Brausebestandteile bestehenden pulverförmigen oder körnigen Gemisches in einem geschlossenen System in Vakuum, wobei die Säure gegebenenfalls nach einer Vorbehandlung bei erhöhter Temperatur mit der nötigen Menge an Hydrogencarbonat und/oder Carbonat vermischt wird und man hierauf dieses Gemisch einer Vakuumbehandlung bei einer Temperatur von 30 von bis 100 °C, vorzugsweise 40 bis 80 °C, bei welcher es mit einem polaren Lösungsmittel wie Wasser, Methanol, Äthanol oder Gemischen hievon versetzt wird, aussetzt, wonach die erhaltenen Aggregate zur gewünschten Teilchengrösse zerkleinert, gegebenenfalls mit den gewünschten Zusatzstoffen versehen und gegebenenfalls tablettiertwerden, dadurch gekennzeichnet, dass zur Passivierung der Oberfläche von zumindest einer der Reaktionskomponenten bzw. zur Überführung derselben in einen Zustand hoher Reaktionsträgheit während der Vakuumbehandlung eine dosierte Menge von bis zu 7 Masse-% bezogen auf die Masse des zu behandelnden Gemisches des polaren Lösungsmittels zum Gemisch hinzufügt, die durch die $CO_2$-Entwicklung ab dem Lösungsmittelzusatz während der Reaktion bis zum Erreichen von max. 1 000 mbar verursachte Druckdifferenz feststellt, aus dieser Druckdifferenz das Volumen und die Masse des freigesetzten $CO_2$ ermittelt und die Wärmebehandlung jeweils nach Schnelltrocknung des Gemisches so oft wiederholt, bis die durch deutliche Verlangsamung der Reaktion bzw. verminderte Gasentwicklung angezeigte Oberflächenpassivierung erreicht ist.

2. Verfahren nach Anspruch 1, dadurch gekennzeichnet, dass als polares Lösungsmittel Wasser eingesetzt wird.

3. Verfahren nach Anspruch 1, dadurch gekennzeichnet, dass als polares Lösungsmittel Alkohole oder Alkohol-Wasser-Gemische, die durch unpolare Lösungsmittel getrennt sind, eingesetzt werden.

4. Verfahren nach Anspruch 3, dadurch gekennzeichnet, dass als unpolare Lösungsmittel Chlorkohlenwasserstoffe oder chlorierte Chlorkohlenwasserstoffe eingesetzt werden.

**Claims**

1. Procedure for the manufacture of effervescent granules that can possibly be processed into effervescent tablets by heat treatment, at a temperature between 30 °C and 100 °C, of a powdered or granular mixture consisting of an acid or acid carbonate and/or a carbonate as effervescent components in a closed system under vacuum, according to which procedure the acid, possibly after a preliminary treatment at a high temperature, is mixed with a required quantity of acid carbonate and/or carbonate and such mixture is then exposed to a treatment under vacuum at a temperature from 30 °C up to 100 °C, but preferably from 40 °C to 80 °C, at which the mixture is mixed with a polar solvent such as water, methanol, ethanol or mixtures of such solvents, after which step the aggregates thus obtained are crushed into particles of the required size and are possibly provided with desired additives and possibly reduced into tablets, such procedure being characterized by the fact that, for the passivation of the surface of at least one of the components of the reaction and so as to reduce the components to a state of strong inertia to the reaction, there is added to the mixture during the treatment under vacuum a metered quantity of polar solvent up to 7 % of the mass considered as being the mass of the mixture which has to be treated, the difference in pressure caused by development of carbon dioxide through the addition of solvent during the reaction is determined up to a maximum of 1,000 m-bars, the volume and mass of the carbon dioxide liberated are ascertained from this difference in pressure, and the heat treatment is repeated, after rapid drying of the mixture, as many times as are necessary to obtain passivation of the surface as indicated by an evident slowing down of the reaction and by a reduced development of gas.

2. Procedure as claimed in Claim 1, in which water is employed as a polar solvent.

3. Procedure as claimed in Claim 1, in which alcohols or alcohol-water mixtures separated by non-polar solvents are employed as a polar solvent.

4. Procedure as claimed in Claim 3, in which hydrocarbides of chlorine or chlorinated hydrocarbides of chlorine are employed as as non-polar solvents.

**Revendications**

1. Procédé de préparation de granulés effervescents pouvant être transformés, le cas échéant, en comprimés effervescents, par traitement thermique entre 30 et 100 °C du mélange pulvérulent ou granulaire composé d'acide et de bicarbonate et/ou de carbonate comme constituants effervescents, dans un système fermé, sous vide, dans lequel l'acide est mélangé, éventuellement après traitement préalable à température élevée, avec la quantité nécessaire de bicarbonate et/ou de carbonate, et, ensuite, on expose ce mélange à un traitement sous vide à une température de 30 à 100 °C et de préférence de 40 à 80 °C, pendant lequel il est mélangé avec un solvant polaire, tel que l'eau, le méthanol, l'éthanol ou des mélanges de ces solvants, après quoi les agrégats ainsi obtenus sont broyés à la grosseur de particules désirée, éventuellement munis des additifs désirés et éventuellement transformés en comprimés, procédé qui est caractérisé en ce que, pour la passivation de la surface d'au moins l'un des constituants de la réaction ou pour mettre ce constituant dans un état de haute inertie à la réaction pendant le traitement sous vide, on ajoute au mélange une quantité dosée du solvant polaire, jusqu'à 7 % en masse, rapportée à la masse du mélange, on détermine la différence de pression provoquée par le dégagement de $CO_2$ à partir de l'addition de solvant, pendant la réaction, jusqu'à ce qu'on atteigne au maximum 1 000 mbar, à partir de cette différence de pression, on calcule le volume et la masse du $CO_2$ et on répète le traitement thermique, après le séchage rapide du mélange sous vide d'un nombre de reprises suffisant pour qu'on ait atteint la passivation superficielle indiquée par un ralentissement notable de la réaction ou par une diminution notable du dégagement de gaz.

2. Procédé selon la revendication 1, caractérisé en ce qu'on utilise de l'eau comme solvant polaire.

3. Procédé selon la revendication 1, caractérisé en ce qu'on utilise comme solvant polaire des alcools ou mélanges d'alcool et d'eau qui sont préparés par des solvants non polaires.

4. Procédé selon la revendication 3, caractérisé en ce qu'on utilise comme solvants non polaires des chlorhydrocarbures ou des chlorhydrocarbures chlorés.